(19) Europäisches Patentamt
European Patent Office
Office européen des brevets

(11) Publication number: **0 442 266 B1**

(12) # EUROPEAN PATENT SPECIFICATION

(45) Date of publication of patent specification: **28.12.94**

(51) Int. Cl.5: **C07C 255/50**, C09K 19/18, G02F 1/137

(21) Application number: **91100404.2**

(22) Date of filing: **15.01.91**

(54) **Tolan derivatives, liquid crystal compositions containing them, and liquid crystal elements using them.**

(30) Priority: **16.02.90 JP 35692/90**
**07.03.90 JP 55570/90**
**12.04.90 JP 96916/90**
**05.06.90 JP 146766/90**
**06.06.90 JP 148095/90**

(43) Date of publication of application:
**21.08.91 Bulletin 91/34**

(45) Publication of the grant of the patent:
**28.12.94 Bulletin 94/52**

(84) Designated Contracting States:
**CH DE GB LI**

(56) References cited:
**EP-A- 0 377 516**
**DE-A- 2 504 641**
**DE-A- 3 906 038**

(73) Proprietor: **SEIKO EPSON CORPORATION**
**4-1, Nishishinjuku 2-chome**
**Shinjuku-ku**
**Tokyo-to (JP)**

(72) Inventor: **Yamada, Shuhei**
**c/o Seiko Epson Corporation,**
**3-5, Owa 3-chome**
**Suwa-shi,**
**Nagano-ken (JP)**

(74) Representative: **Hoffmann, Eckart et al**
**Patentanwalt,**
**Blumbach & Partner,**
**Bahnhofstrasse 103**
**D-82166 Gräfelfing (DE)**

EP 0 442 266 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel tolan derivatives to be used for a material for electro-optical displays, to liquid crystal compositions containing them, and to liquid crystal elements using them.

Liquid crystal display devices utilize the electro-optical effects of liquid crystals, and the liquid crystal phases which are used therefor include the nematic phase, the cholesteric phase, and the smectic phase. The display system which is most widely used at present is the torsional nematic type (hereinafter referred to as TN) in which the nematic phase is utilized.

Liquid crystal display devices have the following advantages:

1. they can be compact and thin,
2. they have a low driving voltage, and require only low electric power, and
3. they are light-receiving elements which result in little tiredness of the eyes even after use for a long time, and consequently have been hitherto employed in watches, desk-top calculators, audio devices, various measuring instruments, dashboards of automobiles and the like. In particular, recently they have been used in displays having numerous picture elements, such as displays for personal computers and word processors, as well as black-and white or color pocket televisions and the like, which are attracting increasing attention as display devices for replacing the CRT. As described above, liquid crystal display devices have been used in a variety of fields, and the application fields thereof will no doubt increase further in future. Although the characteristics required for liquid crystal materials vary depending on the above factors the following characteristics may be considered basic, necessary, and in dispensable.

1. No coloring, stability against heat and light as well as electrically and chemically.
2. The temperature range for practical use is wide.
3. The electro-optical response speed is high.
4. The driving voltage is low.
5. The rise in the voltage-light transmittance characteri stics is sharp, and the temperature dependence of the threshold value thereof (hereinafter referred to as $V_{th}$) is small.
6. The range of the visual angle is wide.

A number of liquid crystals which satisfy the characteristic 1 are known, however, no liquid crystal compound is known which satisfies the characteristics 2 to 6 as a single component. Thus in order to satisfy these characteristics, a liquid crystal composition in which several kinds of nematic liquid crystal compounds or non-liquid crystal compounds are mixed is used.

These include in order to satisfy the characteristic 2, generally a liquid crystal compound of relatively low molecular weight and having a crystal-nematic transition point (hereinafter referred to as C-N point) or a melting point at room temperature or in the vicinity thereof, and a liquid crystal compound of high molecular weight having the N-I point at 200 °C or more; in order to obtain a liquid crystal composition having a wide temperature range for practical use, a liquid crystal compound having an N-I point as high as possible is necessary.

Alternatively, in order to satisfy characteristic 3 taking account of the following relation between the response speed (hereinafter referred to as $\tau$), the viscosity index (hereinafter referred to as $\eta$), and the cell gap (hereinafter referred to as d):

$$\tau \propto \eta d$$

the response speed must be increased in order to decrease d. Moreover, in practically used cells, in order to prevent generation of interference fringes on the surface of cells, which impair the appearance of cells, the value of $\Delta n \cdot d$ is determined to be a constant and a material having a large $\Delta n$ is consequently used, enabling the value of d to be made small and the response speed to be made high.

In addition, in order to satisfy characteristic 4, the threshold voltage should be decreased. However, the following relation holds between $V_{th}$, the elastic constant (hereinafter referred to as K), and $\Delta\epsilon$:

$$V_{th} \propto (K/\Delta\epsilon)^{1/2}$$

so that in order to decrease $V_{th}$, a liquid crystal compound having a large $\Delta\epsilon$ and a small K is necessary.

The object of the invention is to provide a novel liquid crystal compound with which a liquid crystal composition having a low threshold voltage, a large $\Delta n$ and a wide temperature range for practical use can be obtained by mixing, one, two or more nematic liquid crystal compounds or non-liquid crystal compounds.

The present invention resides in novel compounds represented by the general formula:

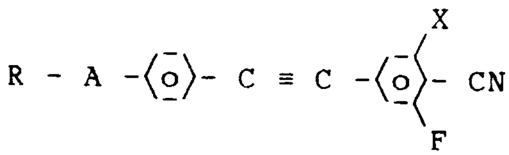 (1)

(wherein R represents a straight chain alkyl group having 1 to 10 carbon atoms, A indicates either

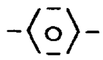 or 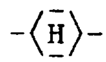

or single bond, and X represents hydrogen or fluorine, provided that the cyclohexane ring has the trans configuration), and liquid crystal compositions containing them.

As shown in the following scheme, the compound (1) of the present invention can be obtained according to following production steps:

Step 1: The compound (2) is reacted in dichloromethane with carbon tetrabromide and triphenylphosphine to obtain the compound (3);

Step 2: The compound (3) is reacted in tetrahydrofuran with butyl lithium to obtain the compound (4);

Step 3: The compound (5) is reacted in acetic acid with iodine chloride to obtain the compound (6);

Step 4: The compound (6) or the compound (7) is reacted in acetic acid with sodium nitrite and sulfuric acid to form a diazonium salt, followed by reacting with sulfuric acid and potassium cyanide to obtain the compound (8);

Step 5: The compound (4) is reacted with the compound (8) in N,N-dimethylformamide in the presence of bis(triphenylphosphine) palladium (II) chloride, cuprous iodide, and diethylamine to obtain the compound (1).

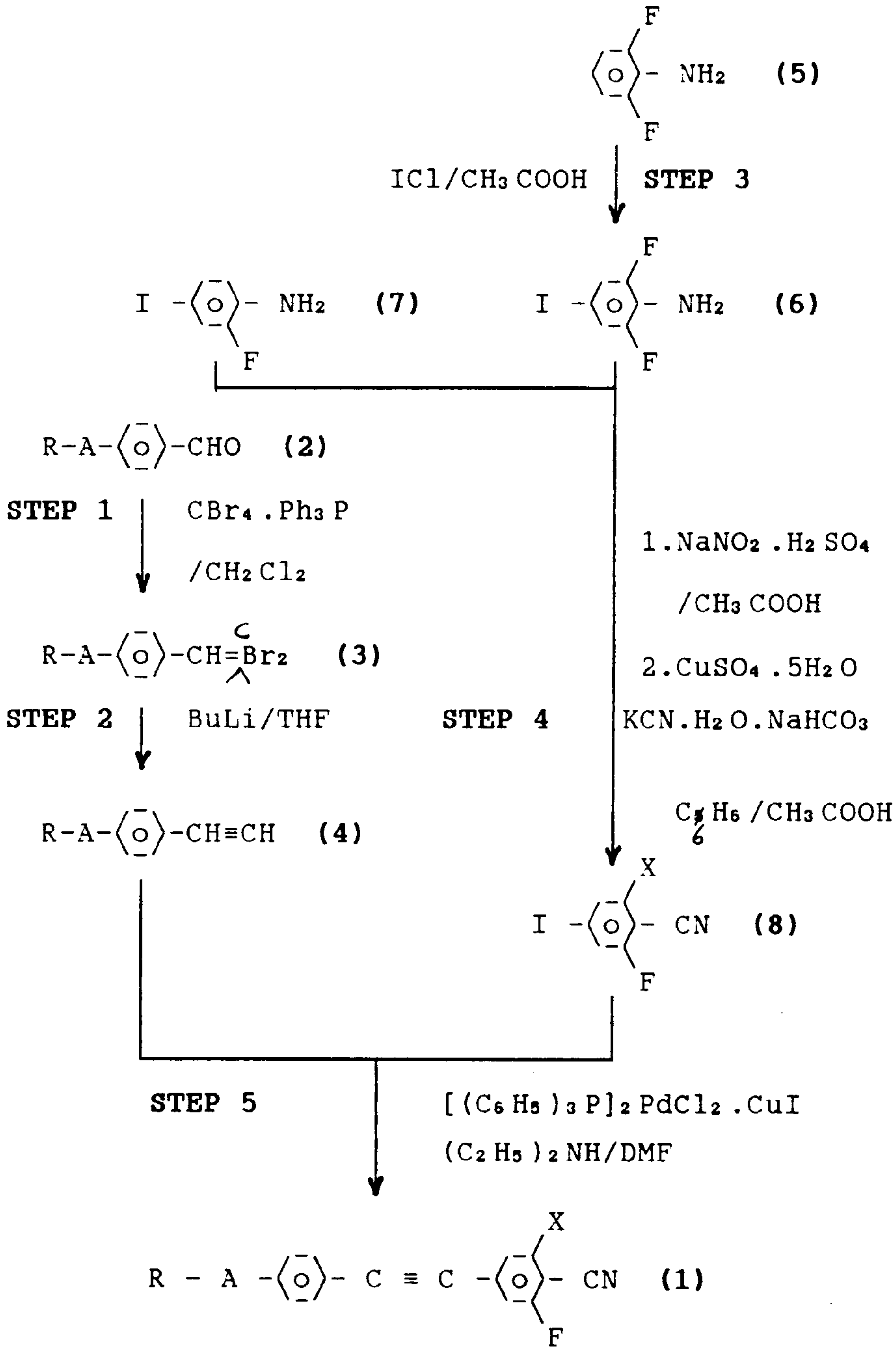


The Figure shows a liquid crystal element used in the examples of the present invention, wherein 1 and 2 are glass substrate, 3 is an electrode, 4 is an orientations-regulating layer, 5 is a polarizing plate and 6 is a sealing member.

The present invention will be further explained in detail with the aid of the following examples hereinafter.

Example 1 (Synthesis of the compound 1-a)

Production of 4-propyl-3'-fluoro-4'-cyanotolan:

Step 1: In 180 ml of dichloromethane 60 g of carbon tetrabromide was dissolved, and at 0 ° C or less 93 g of triphenylphosphine was added. Thereafter 22 g of 4-propylbenzaldehyde was dissolved in 150 ml of dichloromethane, which was added dropwise over a period of one hour. Agitation was then carried out at room temperature for one hour. After completion of the reaction, dichloromethane was distilled off, and 500 ml of hexane was added to the residue. The crystals were filtrated, and then washed with hexane three times. Hexane in the filtrate was distilled off and to the residue a small amount of hexane was added, the crystals were filtrated, and hexane in the filtrate was distilled off. The residue was subjected to vacuum distillation (120 to 130 ° C/4 mmHg) to obtain 27 g of 4-propyl-$\beta$,$\beta$-dibromostyrene.

Step 2: In 44 ml of tetrahydrofuran 27 g of 4-propyl-$\beta$,$\beta$-dibromostyrene was dissolved under nitrogen flow to cool to -78 ° C. 200 ml of a butyl lithium hexane solution (1.6 mol/l) was added dropwise over a period of one hour. Agitation was then carried out at room temperature for one and a half hour. After completion of the reaction, 200 ml of water was added, extraction was carried out with chloroform, and washing was performed three times with water. Chloroform was distilled off, after which vacuum distillation (70 to 75 ° C/20 mmHg) was carried out to obtain 3.5 g of 4-propylphenylacetylene.

Step 4: 27 g of sodium nitrite was dissolved in 205 ml of sulfuric acid and allowed to cool to 10 ° C or less, and then 238 ml of acetic acid was added; at 20 to 25 ° C 65 g of 2-fluoro-4-iodonaniline was added and then agitation was carried out for one hour. 83 g of copper sulfate pentahydrate was dissolved in 205 ml of water, to which was added 140 g of ice. Furthermore a solution in which 88 g of potassium cyanide was dissolved in 200 ml of water, 500 g of sodium bicarbonate, and 200 ml of benzene were added. A diazonium salt sulfuric acid solution was added thereto. After agitation at room temperature for 3 hours, a sodium hydroxide aqueous solution was added to dissolve crystals. After extraction with chloroform, washing was carried out three times with a 10% sodium hydroxide solution and with water. After distilling off the chloroform, the residue was extracted with hexane, and the hexane then distilled off. The residue was subjected to vacuum distillation (95 to 100 ° C/15 mmHg), and recrystallization was then carried out to obtain 20 g of 1-cyano-2-fluoro-4-iodobenzene.

Step 5: In 14 ml of N,N-dimethylformamide 2.1 g of 1-cyano-2-fluoro-4-iodobenzene was dissolved to which were added 3 mg of bis(triphenylphosphine) palladium (II) chloride and 13 mg of cuprous iodide. Then, 1.2 g of 4-propylphenylacetylene obtained in step 2 was added, after which a reaction was carried out at 50 to 60 ° C for 0.5 hour. After completion of the reaction, 35 ml of 9% hydrochloric acid was added to the reaction solution, and extraction was carried out with chloroform, followed by washing with water three times and distilling off the chloroform. The residue was purified by silica gel-chloroform column chromatography, after which recrystallization was carried out with a mixed solvent of acetone and methanol to obtain 0.5 g of 4-propyl-3'-fluoro-4'-cyanotolan. The melting point of this compound (hereinafter referred to as C-I point) was 67.2 ° C.

The following compounds were synthesized by the same method:

4-methyl-3'-fluoro-4'-cyanotolan,
4-ethyl-3'-fluoro-4'-cyanotolan,
4-butyl-3'-fluoro-4'-cyanotolan,
4-pentyl-3'-fluoro-4'-cyanotolan,
4-hexyl-3'-fluoro-4'-cyanotolan (C-I point of 63.8 ° C),
4-heptyl-3'-fluoro-4'-cyanotolan,
4-octyl-3'-fluoro-4'-cyanotolan,
4-nonyl-3'-fluoro-4'-cyanotolan,
4-decyl-3'-fluoro-4'-cyanotolan.

Example 2 (Synthesis of the compound 1-b)

Production of 4-propyl-3',5'-difluoro-4'-cyanotolan:

Step 1: In 180 ml of dichloromethane 60 g of carbon tetrabromide was dissolved and at 0 ° C or less 93 g of triphenylphosphine was added. Then 22 g of 4-propylbenzaldehyde was dissolved in 150 ml of dichloromethane, which was added dropwise over a period of one hour. Agitation was then carried out at room temperature for one hour. After completion of the reaction, dichloromethane was distilled off, and 500 ml of hexane was added to the residue. The crystals were filtrated and further washed with hexane

three times. The hexane in the filtrate was distilled off, and a small amount of hexane was added to the residue, the crystals were filtrated, and the hexane in the filtrate was distilled off. The residue was subjected to vacuum distillation (120 to 130 ° C/4 mmHg) to obtain 27 g of 4-propyl-$\beta$,$\beta$-dibromostyrene.

Step 2: In 44 ml of tetrahydrofuran 27 g of 4-propyl-$\beta$,$\beta$-dibromostyrene was dissolved under nitrogen flow to cool to -78 ° C. 200 ml of a butyl lithium hexane solution (1.6 mol/l) was added dropwise thereto over a period of one hour. Then, agitation was carried out at room temperature for one and a half hour. After completion of the reaction, 200 ml of water was added, extraction was carried out with chloroform, and washing was performed three times with water. The chloroform was distilled off, after which vacuum distillation (70 to 75 ° C/20 mmHg) was carried out to obtain 3.5 g of 4-propylphenylacetylene.

Step 3: 60 g of 2,6-difluoroaniline was dissolved in 180 ml of acetic acid. A solution in which 75 g of iodine chloride was dissolved in 48 ml of acetic acid was added dropwise thereto, and then agitation was carried out at 80 ° C for 2 hours. The reaction solution was poured into water, the precipitated crystals were filtrated, and washing was carried out with water. Recrystallization was performed with methanol, vacuum distillation (125 ° C/22 mmHg) was carried out, and recrystallization was performed again with methanol to obtain 57 g of 2,6-difluoro-4-iodoaniline.

Step 4: 12 g of sodium nitrite was dissolved in 91 ml of sulfuric acid and allowed to cool to 10 ° C or less, and then 100 ml of acetic acid was added; at 20 to 25 ° C 39 g of 2,6-difluoro-4-iodoaniline was then added and agitation was carried out for one hour. 37 g of copper sulfate pentahydrate was dissolved in 91 ml of water, to which was added 62 g of ice. Furthermore a solution of 39 g of potassium cyanide in 92 ml of water, 221 g of sodium bicarbonate, and 91 ml of benzene were then added. A diazonium salt sulfuric acid solution was added thereto. After agitation at room temperature for 3 hours, a sodium hydroxide aqueous solution was added to dissolve the crystals. After extraction with chloroform, washing was carried out three times with a 10% sodium hydroxide solution and water. After distilling off the chloroform, the residue was extracted with hexane, and hexane was distilled off. The residue was subjected to recrystallization with methanol to obtain 6.7 g of 1,3-difluoro-2-cyano-5-iodobenzene.

Step 5: In 35 ml of N,N-dimethylformamide 5.6 g of 1,3-difluoro-2-cyano-5-iodobenzene was dissolved, to which were added 7.3 mg of bis(triphenylphosphine) palladium (II) chloride and 34 mg of cuprous iodide. Then, 3 g of 4-propylphenylacetylene obtained in step 2 was added, after which a reaction was carried out at 50 to 60 ° C for 0.5 hour. After completion of the reaction, 35 ml of 9% hydrochloric acid was added to the reaction solution, and extraction was carried out with chloroform, followed by washing with water three times and distilling off the chloroform. The residue was purified by silica gel-chloroform column chromatography, after which recrystallization was carried out with a mixed solvent of acetone and methanol to obtain 1.6 g of 4-propyl-3',5'-difluoro-4'-cyanotolan. The melting point of this compound was (CI-point) 83,3 ° C.

The following compounds were synthesized by means of the same method:

4-methyl-3',5'-difluoro-4'-cyanotolan,
4-ethyl-3',5'-difluoro-4'-cyanotolan,
4-butyl-3',5'-difluoro-4'-cyanotolan (C-I point of 69.5 ° C),
4-pentyl-3',5'-difluoro-4'-cyanotolan,
4-hexyl-3',5'-difluoro-4'-cyanotolan (C-I point of 50.2 ° C),
4-heptyl-3',5'-difluoro-4'-cyanotolan,
4-octyl-3',5'-difluoro-4'-cyanotolan,
4-nonyl-3',5'-difluoro-4'-cyanotolan,
4-decyl-3',5'-difluoro-4'-cyanotolan.

Example 3 (Synthesis of the compound 1-c)

Production of 4-(4''-butylphenyl)-3'-fluoro-4'-cyanotolan:

Step 1: In 185 ml of dichloromethane 60 g of carbon tetrabromide was dissolved and at 0 ° C or less 100 g of triphenylphosphine was added. Then 22 g of 4'-butyl-4-biphenylaldehyde was dissolved in 93 ml of dichloromethane, which was added dropwise over a period of one hour. Agitation was then carried out at room temperature for one hour. After completion of the reaction, dichloromethane was distilled off, and 500 ml of hexane was added to the residue. The crystals were filtrated, and further washed with hexane three times. The hexane in the filtrate was distilled off, a small amount of hexane added to the residue, the crystals filtrated, and the hexane in the filtrate distilled off. The residue was subjected to recrystallization with hexane to obtain 9.6 g of 4-(4'-butylphenyl)-$\beta$,$\beta$-dibromostyrene.

Step 2: In 50 ml of tetrahydrofuran 9.6 g of 4-(4'-butylphenyl)-$\beta$,$\beta$-dibromostyrene was dissolved under nitrogen flow and allowed to cool to -78 °C. 100 ml of a butyl lithium hexane solution (1.6 mol/l) was added dropwise thereto over a period of one hour. Agitation was then carried out at room temperature for one and a half hour. After completion of the reaction, 500 ml of water was added, extraction was carried out with chloroform, and washing was performed three times with water. The chloroform was distilled off, after which the solvent was completely removed to obtain 5 g of 4'-butyl-4-biphenylacetylene.

Step 4: 27 g of sodium nitrite was dissolved in 205 ml of sulfuric acid and allowed to cool to 10 °C or less; 238 ml of acetic acid was then added and at 20 to 25 °C 65 g of 2-fluoro-4-iodoaniline was added, and agitation then carried out for one hour. 82.5 g of copper sulfate pentahydrate was dissolved in 20.5 ml of water, to which 140 g of ice was added. Furthermore a solution of 88 g of potassium cyanide in 207 ml of water, 500 g of sodium bicarbonate, and 206 ml of benzene were added. A diazonium salt sulfuric acid solution was added thereto. After agitation at room temperature for 3 hours, a sodium hydroxide aqueous solution was added to dissolve the crystals. After extraction with chloroform, washing was carried out three times with a 10% sodium hydroxide solution and water. After distilling off the chloroform, the residue was subjected to vacuum distillation (95 to 100 °C/15 mmHg), and recrystallization was further carried out with methanol to obtain 20 g of 1-cyano-2-fluoro-4-iodobenzene.

Step 5: In 15 ml of N,N-dimethylformamide 2.1 g of 1-cyano-2-fluoro-4-iodobenzene was dissolved, to which were added 3 mg of bis(triphenylphosphine) palladium (II) chloride and 14 mg of cuprous iodide. Then, 2 g of 4'-butyl-4-biphenylacetylene which was obtained in step 2 was added, after which a reaction was carried out at 50 to 60 °C for 0.5 hour. After completion of the reaction, 35 ml of 9% hydrochloric acid was added to the reaction solution, and extraction was carried out with chloroform, followed by washing with water three times and distilling off the chloroform. The residue was purified by silica gel-chloroform column chromatography, after which recrystallization was carried out with a mixed solvent of acetone and methanol to obtain 0.4 g of 4-(4''-butylphenyl)-3'-fluoro-4'-cyanotolan. The C-N point of this compound was 99.4 °C, and then N-I point was 214.5 °C.

The following compounds were synthesized by the same method:

4-(4''-methylphenyl)-3'-fluoro-4'-cyanotolan,
4-(4''-ethylphenyl)-3'-fluoro-4'-cyanotolan,
4-(4''-propylphenyl)-3'-fluoro-4'-cyanotolan (C-N point of 129.4 °C, N-I point of 232.8 °C),
4-(4''-pentylphenyl)-3'-fluoro-4'-cyanotolan,
4-(4''-hexylphenyl)-3'-fluoro-4'-cyanotolan (C-N point of 64.2 °C, N-I point of 198.4 °C),
4-(4''-heptylphenyl)-3'-fluoro-4'-cyanotolan (C-N point of 72.5 °C, N-I point of 190.3 °C),
4-(4''-octylphenyl)-3'-fluoro-4'-cyanotolan,
4-(4''-nonylphenyl)-3'-fluoro-4'-cyanotolan,
4-(4''-decylphenyl)-3'-fluoro-4'-cyanotolan.

Example 4 (Synthesis of the compound 1-d)

Production of 4-(4''-butylphenyl)-3',5'-difluoro-4'-cyanotolan:

Step 1: In 185 ml of dichloromethane 60 g of carbon tetrabromide was dissolved, and at 0 °C or less 100 g of triphenylphosphine was added. Then 22 g of 4'-butyl-4-biphenylaldehyde was dissolved in 93 ml of dichloromethane, which was added dropwise over a period of one hour. Agitation was then carried out at room temperature for one hour. After completion of the reaction, dichloromethane was distilled off, and 500 ml of hexane was added to the residue. The crystals were filtrated and further washed with hexane three times. The hexane in the filtrate was distilled off, a small amount of hexane was added to the residue, the crystals were filtrated and the hexane in the filtrate was distilled off. The residue was subjected to recrystallization with hexane to obtain 9.6 g of 4-(4'-butylphenyl)-$\beta$,$\beta$-dibromostyrene.

Step 2: In 50 ml of tetrahydrofuran 9.6 g of 4-(4'-butylphenyl)-$\beta$,$\beta$-dibromostyrene was dissolved under nitrogen flow and allowed to cool to - 78 °C. 100 ml of a butyl lithium hexane solution (1.6 mol/l) was added dropwise thereto over a period of one hour. Agitation was then carried out at room temperature for one and a half hour. After completion of the reaction, 500 ml of water was added, extraction was carried out with chloroform, and washing was performed three times with water. The chloroform was distilled off, after which the solvent was completely removed to obtain 5 g of 4'-butyl-4-biphenylacetylene.

Step 3: By the same method as Example 2, 2,6-difluoro-4-iodoaniline was obtained.

Step 4: By the same method as Example 2, 1,3-difluoro-2-cyano-5-iodobenzenewas obtained.

Step 5: In 19 ml of N,N-dimethylformamide 3 g of 1,3-difluoro-2-cyano-5-iodobenzene was dissolved, to which were added 4 mg of bis(triphenylphosphine) palladium (II) chloride and 18 mg of cuprous iodide.

Then, 3 g of 4'-butyl-4-biphenylacetylene which was obtained in step 2 was added, after which the reaction was carried out at 50 to 60 °C for 0,5 hour. After completion of the reaction, 35 ml of 9% hydrochloric acid was added to the reaction solution, and extraction was carried out with chloroform, followed by washing with water three times and distilling of the chloroform. The residue was purified by silica gel-chloroform column chromatography, after which recrystallization was carried out with a mixed solvent of acetone and methanol to obtain 0.4 g of 4-(4''-butylphenyl)-3',5'-difluoro-4'-cyanotolan. The C-N point of this compound was 101.0 °C and the N-I point was 165.7 °C.

The following compounds were synthesized by the same method:

4-(4''-methylphenyl)-3',5'-difluoro-4'-cyanotolan,
4-(4''-ethylphenyl)-3',5'-difluoro-4'-cyanotolan,
4-(4''-propylphenyl)-3',5'-difluoro-4'-cyanotolan (C-N point of 142.8 °C, N-I point of 182.8 °C),
4-(4''-pentylphenyl)-3',5'-difluoro-4'-cyanotolan,
4-(4''-hexylphenyl)-3',5'-difluoro-4'-cyanotolan (C-N point of 52.0 °C, N-I point of 152.5 °C),
4-(4''-heptylphenyl)-3',5'-difluoro-4'-cyanotolan (C-N point of 60.9 °C, N-I point of 149.2 °C),
4-(4''-octylphenyl)-3',5'-difluoro-4'-cyanotolan,
4-(4''-nonylphenyl)-3',5'-difluoro-4'-cyanotolan,
4-(4''-decylphenyl)-3',5'-difluoro-4'-cyanotolan.

Example 5 (Synthesis of the compound 1-d

Production of 4-(trans-4''-ethylcyclohexyl)-3'-fluoro-4'-cyanotolan:

Step 1: In 450 ml of dichloromethane 148 g of carbon tetrabromide was dissolved and at 0 °C or less 93 g of triphenylphosphine added. Then 48.6 g of 4-(tans-4'-ethylcyclohexyl) benzaldehyde was dissolved in 225 ml of dichloromethane, which was added dropwise over a period of one hour. Agitation was then carried out at room temperature for one hour. After completion of the reaction, dichloromethane was distilled off, and 500 ml of hexane was added to the residue. The crystals were filtrated, and further washed with hexane three times. The hexane in the filtrate was distilled off, a small amount of hexane was added to the residue, the crystals were filtrated, and hexane in the filtrate was distilled off. The residue was subjected to recrystallization with hexane to obtain 52 g of 4-(trans-4'-ethylcyclohexyl)-β,β-dibromostyrene.

Step 2: In 250 ml of tetrahydrofuran 52 g of 4-(trans-4'-ethylcyclohexyl)-β,β-dibromostyrene was dissolved under nitrogen flow and allowed to cool to -78 °C. 300 ml of a butyl lithium hexane solution (1.6 mol/l) was added dropwise thereto over a period of one hour. Agitation was then carried out at room temperature for one and a half hour. After completion of the reaction, 500 ml of water was added, extraction was carried out with chloroform, and washing was performed three times with water. The chloroform was distilled off, after which vacuum distillation (110 to 120 °C/30 mmHg) was carried out to obtain 20 g of 4-(trans-4'-ethylcyclohexyl)acetylene.

Step 4: 27 g of sodium nitrite was dissolved in 205 ml of sulfuric acid and allowed to cool to 10 °C or less, and then 238 ml of acetic acid was added; at 20 to 25 °C 65 g of 2-fluoro-4-iodonaniline was added, and then agitation was carried out for one hour. 82.5 g of copper sulfate pentahydrate was dissolved in 20.5 ml of water, to which was added 140 g of ice. Furthermore a solution of 88 g of potassium cyanide in 207 ml of water, 500 g of sodium bicarbonate, and 206 ml of benzene were added. A diazonium salt sulfuric acid solution was added thereto. After agitation at room temperature for 3 hours, a sodium hydroxide aqueous solution was added to dissolve the crystals. After extraction with chloroform, washing was carried out three times with a 10% sodium hydroxide solution and water. After distilling off the chloroform, the residue was subjected to vacuum distillation (95 to 100 °C/15 mmHg), and recrystallization was further carried out with methanol to obtain 20 g of 1-cyano-2-fluoro-4-iodobenzene.

Step 5: In 25 ml of N,N-dimethylformamide 3.8 g of 1-cyano-2-fluoro-4-iodobenzene was dissolved to which were added 5.3 mg of bis(triphenylphosphine)palladium (II) chloride and 25 mg of cuprous iodide. Then 3.2 g of 4-(trans-4'-ethylcyclohexyl)acetylene obtained in step 2 was added, after which the reaction was performed at 50 to 60 °C for 0.5 hour. After completion of the reaction, 3.5 ml of 9% hydrochloric acid was added to the reaction solution, and extraction was carried out with chloroform, followed by washing with water three times to distill off the chloroform. The residue was purified by silica gel-chloroform column chromatography, after which recrystallization was carried out with a mixed solvent of acetone and methanol to obtain 0.8 g of 4-(trans-4''-ethylcyclohexyl)-3'-fluoro-4'-cyanotolan. The C-N point of this compound was 129.3 °C, and the N-I point was 201.3 °C.

The following compounds were synthesized by means of the same method:
4-(trans-4''-methylcyclohexyl)-3'-fluoro-4'-cyanotolan,
4-(trans-4''-propylcyclohexyl)-3'-fluoro-4'-cyanotolan (C-N point of 113.3°C, N-I point of 213.8°C),
4-(trans-4''-butylcyclohexyl)-3'-fluoro-4'-cyanotolan,
4-(trans-4''-pentylcyclohexyl)-3'-fluoro-4'-cyanotolan,
4-(trans-4''-hexylcyclohexyl)-3'-fluoro-4'-cyanotolan,
4-(trans-4''-heptylcyclohexyl)-3'-fluoro-4'-cyanotolan,
4-(trans-4''-octylcyclohexyl)-3'-fluoro-4'-cyanotolan,
4-(trans-4''-nonylcyclohexyl)-3'-fluoro-4'-cyanotolan,
4-(trans-4''-decylcyclohexyl)-3'-fluoro-4'-cyanotolan.

Example 6 (Synthesis of the compound 1-f)

Production of 4(trans-4''-propylcyclohexyl)-3',5'-difluoro-4'-cyanotolan:

Step 1: In 82 ml of dichloromethane 26.9 g of carbon tetrabromide was dissolved and at 0°C or less 42.6 g of triphenylphosphine added. Then 10 g of 4-(trans-4'-propylcyclohexyl)benzaldehyde was dissolved in 41 ml of dichloromethane, which was added dropwise over a period of on hour. Agitation was then carried out at room temperature for one hour. After completion of the reaction, dichloromethane was distilled off, and 500 ml of hexane was added to the residue. The crystals were filtrated, and further washed with hexane three times. Hexane in the filtrate was distilled off, to the residue a small amount of hexane added, the crystals filtrated, and hexane in the filtrate distilled off. The residue was subjected to recrystallization with hexane to obtain 16 g of 4-(trans-4'-propylcyclohexyl)-β,β-dibromostyrene.
Step 2: In 76 ml of tetrahydrofuran 16 g of 4-(trans-4'-propylcyclohexyl)-β,β-dibromostyrene was dissolved under nitrogen flow and allowed to cool to -78°C. 100 ml of a butyl lithium hexane solution (1.6 mol/l) was added dropwise thereto over a period of one hour. Agitation was then carried out at room temperature for one and a half hour. After completion of the reaction, 200 ml of water was added, extraction was carried out with chloroform, and washing was performed three times with water. Chloroform was distilled off, after which recrystallization was carried out with methanol to obtain 2.45 g of 4-(trans-4'-propylcyclohexyl)acetylene.
Step 3: By the same method as Example 2, 2,6-difluoro-4-iodoaniline was obtained.
Step 4: By the same method as Example 2, 1,3-difluoro-2-cyano-5-iodobenzenewas obtained.
Step 5: In 15 ml of N,N-dimethylformamide 2.5 g of1,3-difluoro-2-cyano-5-iodobenzene was dissolved to which were added 3.3 mg of bis(triphenylphosphine) palladium (II) chloride and 15 mg of cuprous iodide. Then, 2.45 g of 4-(trans-4'-propylcyclohexyl)acetylene obtained in step 2 was added, after which the reaction was carried out at 50 to 60°C for 0.5 hours. After completion of the reaction, 29 ml of 9% hydrochloric acid was added to the reaction solution, and extraction was carried out with chloroform, followed by washing with water and washing twice with 10% hydrochloric acid, 5% hydrogencarbonate aqueous solution, and water, in that order. The chloroform was then distilled off, and purification was carried out with a column chromatography, after which vacuum distillation (150 to 160°C/3 mmHg) was performed, and further recrystallization was carried out with a mixed solvent of acetone and methanol to obtain 0.3 g of 4-(trans-4''-propylcyclohexyl)-3',5-difluoro-4'-cyanotolan. The C-N point of this compound was 105.2°C, and the N-I point was 175.0°C.

The following compounds were synthesized by the same method:
4-(trans-4''-methylcyclohexyl)-3',5'-difluoro-4'-cyanotolan,
4-(trans-4''-ethylcyclohexyl)-3',5'-difluoro-4'-cyanotolan (C-N point of 121.8°C, N-I point of 150.4°C,
4-(trans-4''-butylcyclohexyl)-3',5'-difluoro-4'-cyanotolan (C-N point of 146.2°C, N-I point of 170.4°C),
4-(trans-4''-pentylcyclohexyl)-3',5'-difluoro-4'-cyanotolan (C-N point of 99.5 °C N-I point of 172.4°C),
4-(trans-4''-hexylcyclohexyl)-3',5'-difluoro-4'-cyanotolan,
4-(trans-4''-heptylcyclohexyl)3',5'-difluoro-4'-cyanotolan,
4-(trans-4''-octylcyclohexyl)-3',5'-difluoro-4'-cyanotolan,
4-(trans-4''-nonylcyclohexyl)-3',5'-difluoro-4'-cyanotolan,
4-(trans-4''-decylcyclohexyl)-3',5'-difluoro-4'-cyanotolan,
4-(trans-4''-propylcyclohexyl)-3',5'-difluoro-4'-cyanotolan.

Examples 7 to 12 (use examples)

Using a commercially available mixed liquid crystal ZLI-1565 (a product of Merck Co., Ltd., N-I point of 89.3°C) as a base liquid crystal, 4-butylbenzoic acid 4'-cyanophenyl ester being generally used to decrease Vth, and 4-pentyl-4"-cyanoterphenyl being used to increase the N-I point and having a large Δn were compared with the compounds of the present invention with respect to their characteristics.

Table 1

| | ZLI-1565 | A | B | 1-a | 1-b | 1-c | 1-d | 1-e | 1-f |
|---|---|---|---|---|---|---|---|---|---|
| Comparative test A | 90pbw | 10pbw | | | | | | | |
| Comparative test B | 90pbw | | 10pbw | | | | | | |
| Example 7 | 90pbw | | | 10pbw | | | | | |
| Example 8 | 90pbw | | | | 10pbw | | | | |
| Example 9 | 90pbw | | | | | 10pbw | | | |
| Example 10 | 90pbw | | | | | | 10pbw | | |
| Example 11 | 90pbw | | | | | | | 10pbw | |
| Example 12 | 90pbw | | | | | | | | 10pbw |

A: 4-butylbenzoic acid 4'-cyanophenyl ester
B: 4-pentyl-4"-cyanoterphenyl

1-a: 4-propyl-3'-fluoro-4'-cyanotolan
1-b: 4-propyl-3',5'-difluoro-4'-cyanotolan
1-c: 4-(4"-butylphenyl)-3'-fluoro-4'-cyanotolan
1-d: 4-(4"-butylphenyl)-3',5'-difluoro-4'-cyanotolan
1-e: 4-(trans-4"-ethylcyclohexyl)-3'-fluoro-4'-cyanotolan
1-f: 4-(trans-4"-ethylcyclohexyl)-3',5'-difluoro-4'-cyanotolan
pbw: parts by weight

Compositions according to Table 1 for comparative tests A and B, and examples 7 to 12 were prepared. Then, as shown in Fig. 1, on glass substrates 1 and 2 electrodes 3 composed of transparent conductive films (for example, ITO films) were formed to which orientation materials comprising polyimide and the like, were applied. The materials were subjected to a rubbing treatment to form orientation-regulating layers 4; then the glass substrate 1 and 2 were arranged opposite each other via sealing members 6 to make a liquid crystal cell, and the above mentioned liquid crystal compositions were poured between the glass substrate.

The cell had a thickness of 8 µm and was made as liquid crystal cell of the TN type. The liquid crystal cells thus prepared were subjected to alternating current static driving at 20 °C to measure the voltage-brightness characteristics. The results thereof are shown in Table 2.

In the Table 2, $V_{10}$ is the voltage value to obtain a transmittance of 10%, which is a measured value with a TN cell with a measuring direction $\Theta = 90°$. $\alpha$ and $\beta$ are factors which represent respectively a visual angle characteristic and a threshold characteristic and which are defined as follows:

$\alpha = \Theta 90° V_{50} / \Theta 50° V_{50}$
$\beta = \Theta 90° V_{10} / \Theta 90° V_{90}$

Table 2

| | N-I point | Δn | $V_{10}$ | α | β |
|---|---|---|---|---|---|
| Comparative test A | 83.6 °C | 0.133 | 2.032 | 1.275 | 1.405 |
| Comparative test B | 100.8 °C | 0.149 | 2.415 | 1.292 | 1.425 |
| Example 7 | 80.8 °C | 0.146 | 1.962 | 1.286 | 1.380 |
| Example 8 | 76.5 °C | 0.140 | 1.710 | 1.285 | 1.379 |
| Example 9 | 97.4 °C | 0.158 | 2.200 | 1.289 | 1.398 |
| Example 10 | 93.0 °C | 0.154 | 1.927 | 1.282 | 1.388 |
| Example 11 | 94.8 °C | 0.147 | 2.121 | 1.291 | 1.403 |
| Example 12 | 91.3 °C | 0.144 | 1.895 | 1.302 | 1.397 |

Although in the above mentioned examples a liquid crystal cell of the TN type was used, the same effects are obtained even when a liquid crystal cell of the super twisted nematic type (STN) is employed.

As described above, the compounds of the present invention may be mixed with a general liquid crystal composition, thus achieving the effects that without impairing the electro-optical characteristics (α value and β value) the threshold voltage is greatly decreased and Δn is increased. Moreover, with the compounds of 1-c, 1-d, 1-e, and 1-f a wide temperature range for practical uses is also obtained. In virtue of these points the present invention is very useful as basic constitution component of liquid crystal compositions of a display of the super twisted nematic type, which is the most common type of liquid crystal display devices at present.

## Claims

**1.** Tolan derivatives, characterized in that they are represented by the general formula:

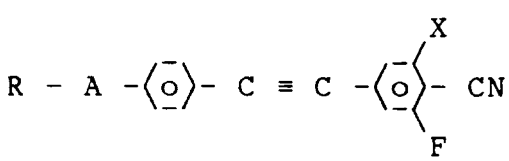

(1)

wherein R indicates a straight chain alkyl group having 1 to 10 carbon atoms, A represents either

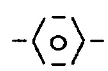

or

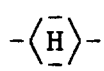

or single bond, and X represents hydrogen or fluorine, provided that the cyclohexane ring has the trans configuration.

**2.** The tolan derivatives according to claim 1, characterized in that they are represented by the general formula:

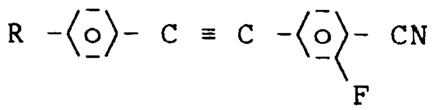

(1-a)

wherein R represents a straight chain alkyl group having 1 to 10 carbon atoms.

**3.** The tolan derivatives according to claim 1, characterized in that they are represented by the general formula:

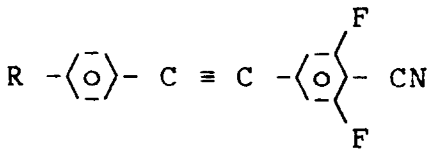

(1-b)

wherein R represents a straight chain alkyl group having 1 to 10 carbon atoms.

**4.** The tolan derivatives according to claim 1, characterized in that they are represented by the general formula:

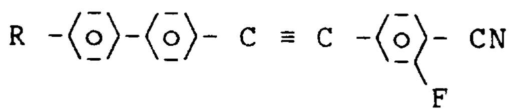

(1-c)

wherein R represents a straight chain alkyl group having 1 to 10 carbon atoms.

**5.** The tolan derivatives according to claim 1, characterized in that they are represented by the general formula:

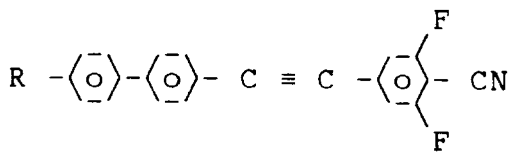

(1-d)

wherein R represents a straight chain alkyl group having 1 to 10 carbon atoms.

**6.** The tolan derivatives according to claim 1, characterized in that they are represented by the general formula:

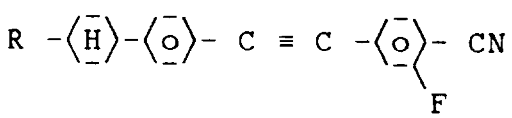

(1-e)

wherein R represents a straight chain alkyl group having 1 to 10 carbon atoms, and the cyclohexane ring has the trans configuration.

**7.** The tolan derivatives according to claim 1, characterized in that they are represented by the general formula:

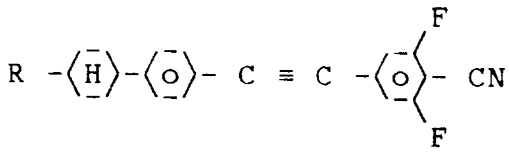 (1-f)

wherein R represents a straight chain alkyl group having 1 to 10 carbon atoms, and the cyclohexane ring has the trans configuration.

8. A liquid crystal composition, characterized in that it contains at least one kind of a tolan derivative represented by the general formula:

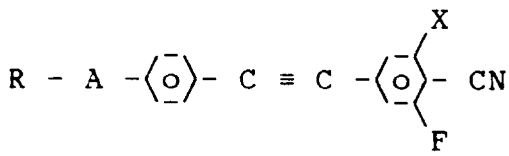 (1)

wherein R indicates a straight chain alkyl group having 1 to 10 carbon atoms, A represents either

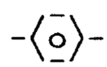 or 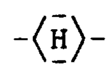

or single bond, and X represents hydrogen or fluorine, provided that the cyclohexane ring has the trans configuration.

9. A liquid crystal element, characterized in that a liquid crystal composition is used which contains at least one kind of a tolan derivative represented by the general formula:

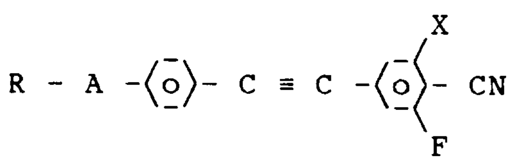 (1)

wherein R indicates a straight chain alkyl group having 1 to 10 carbon atoms, A represents either

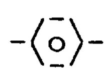 or 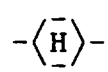

or single bond, and X represents hydrogen or fluorine, provided that the cyclohexane ring has the trans configuration.

**Patentansprüche**

1. Tolanderivate, dadurch gekennzeichnet, daß sie durch die folgende allgemeine Formel dargestellt werden:

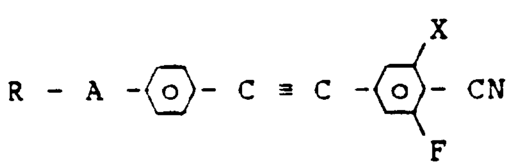 (1)

in der R eine geradkettige Alkylgruppe mit 1 bis 10 Kohlenstoffatomen bezeichnet, A entweder

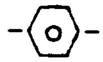 oder 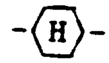

oder eine Einfachbindung darstellt und X Wasserstoff oder Fluor darstellt, mit der Maßgabe, daß der Cyclohexanring die trans-Konfiguration aufweist.

**2.** Tolanderivate nach Anspruch 1, dadurch gekennzeichnet, daß sie durch die folgende allgemeine Formel dargestellt werden:

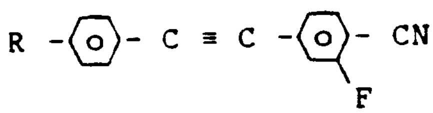 (1-a)

in der R eine geradkettige Alkylgruppe mit 1 bis 10 Kohlenstoffatomen darstellt.

**3.** Tolanderivate nach Anspruch 1, dadurch gekennzeichnet, daß sie durch die folgende allgemeine Formel dargestellt werden:

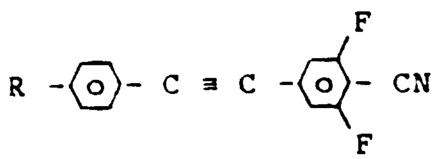 (1-b)

in der R eine geradkettige Alkylgruppe mit 1 bis 10 Kohlenstoffatomen darstellt.

**4.** Tolanderivate nach Anspruch 1, dadurch gekennzeichnet, daß sie durch die folgende allgemeine Formel dargestellt werden:

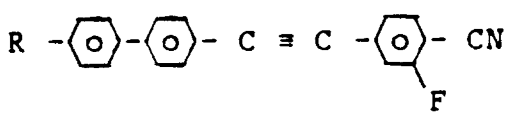 (1-c)

in der R eine geradkettige Alkylgruppe mit 1 bis 10 Kohlenstoffatomen darstellt.

**5.** Tolanderivate nach Anspruch 1, dadurch gekennzeichnet, daß sie durch die folgende allgemeine Formel dargestellt werden:

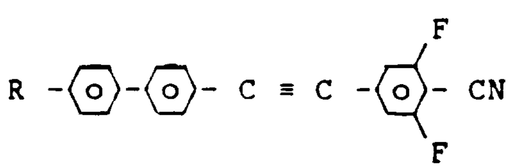 (1-d)

in der R eine geradkettige Alkylgruppe mit 1 bis 10 Kohlenstoffatomen darstellt.

**6.** Tolanderivate nach Anspruch 1, dadurch gekennzeichnet, daß sie durch die folgende allgemeine Formel dargestellt werden:

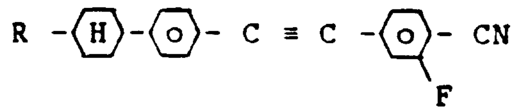

(1-e)

in der R eine geradkettige Alkylgruppe mit 1 bis 10 Kohlenstoffatomen darstellt und der Cyclohexanring die trans-Konfiguration aufweist.

**7.** Tolanderivate nach Anspruch 1, dadurch gekennzeichnet, daß sie durch die folgende allgemeine Formel dargestellt werden:

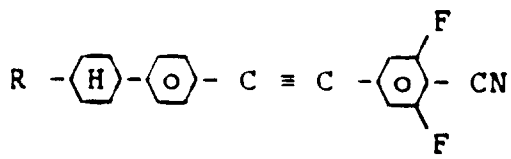

(1-f)

in der R eine geradkettige Alkylgruppe mit 1 bis 10 Kohlenstoffatomen darstellt und der Cyclohexanring dir trans-Konfiguration aufweist.

**8.** Flüssigkristallzusammensetzung, dadurch gekennzeichnet, daß sie mindestens eine Art von Tolanderivat enthält, das durch die folgende allgemeine Formel dargestellt wird:

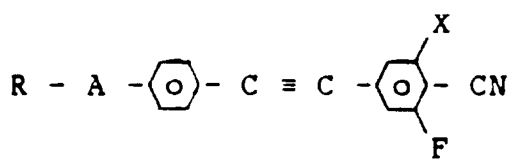

(1)

in der R eine geradkettige Alkylgruppe mit 1 bis 10 Kohlenstoffatomen bezeichnet, A entweder

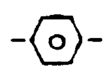 oder 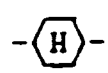

oder eine Einfachbindung darstellt und X Wasserstoff oder Fluor darstellt, mit der Maßgabe, daß der Cyclohexanring die trans-Konfiguration aufweist.

**9.** Flüssigkristallelement, dadurch gekennzeichnet, daß eine Flüssigkristallzusammensetzung verwendet wird, die mindestens eine Art von Tolanderivat enthält, das durch die folgende allgemeine Formel dargestellt wird:

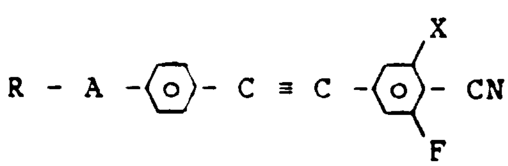

(1)

in der R eine geradkettige Alkylgruppe mit 1 bis 10 Kohlenstoffatomen bezeichnet, A entweder

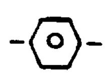 oder 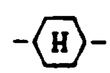

oder eine Einfachbindung darstellt und X Wasserstoff oder Fluor darstellt, mit der Maßgabe, daß der Cyclohexanring die trans-Konfiguration aufweist.

**Revendications**

1. Tolanes, caractérisés en ce qu'ils sont représentés par la formule développée

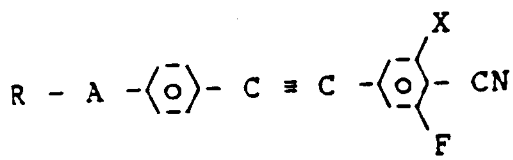

(1)

dans laquelle R indique un groupe alcoyle linéaire de 1 à 10 atomes de carbone, A représente

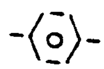

ou

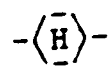

ou une liaison simple et X représente l'hydrogène ou le fluor, pourvu que le cycle cyclohexanique ait la configuration trans.

2. Tolanes suivant la revendication 1, caractérisés en ce qu'ils sont représentés par la formule développée

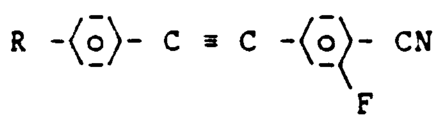

(1-a)

dans laquelle R est un groupe alcoyle linéaire ayant de 1 à 10 atomes de carbone.

3. Tolanes suivant la revendication 1, caractérisés en ce qu'ils sont représentés par la formule développée

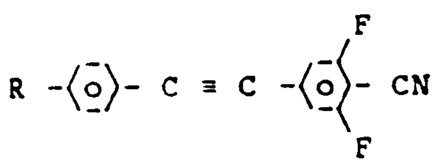

(1-b)

dans laquelle R est un groupe alcoyle linéaire ayant de 1 à 10 atomes de carbone.

4. Tolanes suivant la revendication 1, caractérisés en ce qu'ils sont représentés par la formule développée

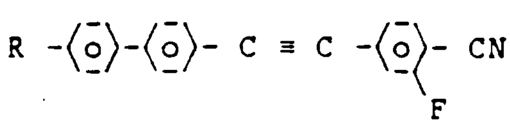

(1-c)

dans laquelle R est un groupe alcoyle linéaire ayant de 1 à 10 atomes de carbone.

**5.** Tolanes suivant la revendication 1, caractérisés en ce qu'ils sont représentés par la formule développée

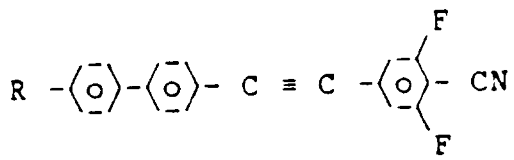

(1-d)

dans laquelle R représente un groupe alcoyle linéaire ayant de 1 à 10 atomes de carbone.

**6.** Tolanes suivant la revendication 1, caractérisés en ce qu'ils sont représentés par la formule développée

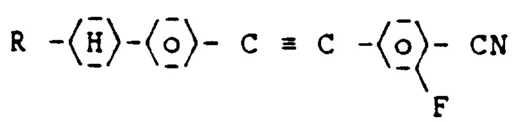

(1-e)

dans laquelle R représente un groupe alcoyle linéaire ayant de 1 à 10 atomes de carbone et le cycle cyclohexanique a la configuration trans.

**7.** Tolanes suivant la revendication 1, caractérisés en ce qu'ils sont représentés par la formule développée

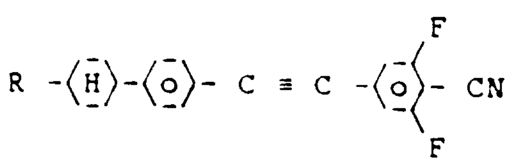

(1-f)

dans laquelle R représente un groupe alcoyle linéaire ayant de 1 à 10 atomes de carbone et le cycle cyclohexanique a la configuration trans.

**8.** Composition de cristal liquide, caractérisée en ce qu'elle contient au moins un type de tolanes représentés par la formule développée

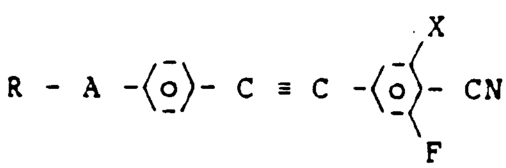

(1)

dans laquelle R indique un groupe alcoyle linéaire ayant de 1 à 10 atomes de carbone, A représente

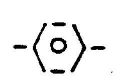

ou

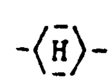

ou une liaison simple et X représente l'hydrogène ou le fluor, pourvu que le cycle cyclohexanique ait la configuration trans.

**9.** Élément de cristal liquide, caractérisé en ce qu'une composition de cristal liquide est utilisée, qui contient au moins un type d'un tolane représenté par la formule développée

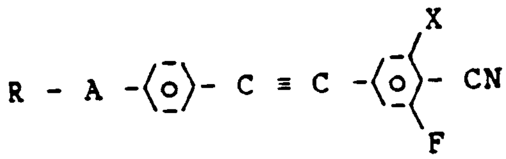

(1)

dans laquelle R indique un groupe alcoyle linéaire ayant de 1 à 10 atomes de carbone, A représente

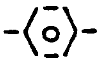

ou

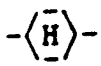

ou une liaison simple et X représente l'hydrogène ou le fluor, pourvu que le cycle cyclohexanique ait la configuration trans.

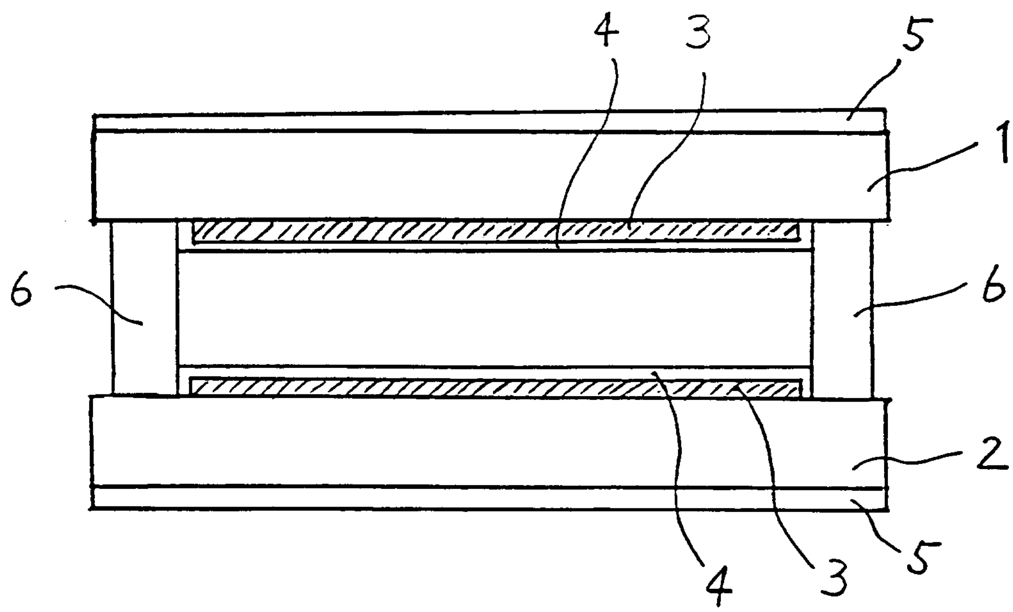
4
3
5
1
6
6
2
4
3
5